# EUROPÄISCHE PATENTSCHRIFT

(11) **EP 0 563 657 B1**
(45) Veröffentlichungstag und Bekanntmachung des Hinweises auf die Patenterteilung: **02.11.1995**
(21) Anmeldenummer: 93104099.2
(22) Anmeldetag: 12.03.1993
(51) Int. Cl.: A61F 2/76

(54) **Verbindungsteil zwischen Beinprothesen-Komponenten**
Connecting element between components of a leg prosthesis
Elément de connexion entre composants d'une prothèse de jambe

(30) Priorität: 01.04.1992 DE 9204448 U
(43) Veröffentlichungstag der Anmeldung: 06.10.1993
(73) Patentinhaber: Otto Bock Orthopädische Industrie Besitz- und Verwaltungs-Kommanditgesellschaft, 37115 Duderstadt (DE)
(72) Erfinder: Hiemisch, Christian, W-3408 Duderstadt (DE)
(74) Vertreter: Gramm, Werner, Prof., Dipl.-Ing.

(56) Entgegenhaltungen:
- CH-A- 499 982
- FR-A- 1 584 288
- FR-A- 2 630 641
- US-A- 4 969 911

## Beschreibung

Die Erfindung betrifft ein Verbindungsteil eines Kugelgelenkes zwischen Beinprothesen-Komponenten mit einem zur Befestigung an der einen Komponente bestimmten Flansch, auf dem ein Justierelement angeordnet ist, das aus einer Kalotte und einer zentrisch auf dieser auf ihre Spitze gestellten vierflächigen Pyramide besteht.

Eine entsprechende Ausführungsform läßt sich der deutschen Patentschrift Nr. 19 22 619 entnehmen. Hier befindet sich das durch die Kalotte und die Pyramide gebildete Jusierelement genau mittig auf dem Befestigungsflansch. Durch die Verstellung von jeweils zwei sich gegenüberliegenden Stellschrauben läßt sich eine Justierung des Kugelgelenkes in nur zwei definierten Ebenen durchführen. Hierdurch läßt sich vorteilhafterweise eine Beinprothese z. B. auf die Belastungslinie einstellen.

Dieser vorbekannte Adapter wird üblicherweise zur Verbindung des prothetischen Ersatzsystems mit dem Prothesenschaft für Unterschenkelstümpfe und kurze bis mittellange Oberschenkelstümpfe eingesetzt. Bei Stumpffehlstellungen z. B. in Beuge- und/oder Abspreizrichtung, die insbesondere bei der ständig wachsenden Gruppe beinamputierter Geriatrie-Patienten zu finden sind, kann es beim Prothesenaufbau unter Umständen notwendig werden, den größten Teil des in jeweils einer Richtung vorhandenen, kontruktiv aber begrenzten Justierbereichs zur Fehlstellungs-Kompensation zu verwenden, so daß der verbleibende Justier-Restbereich für Justierungen nach biomechanischen Kriterien nicht mehr ausreicht.

Der Erfindung liegt die Aufgabe zugrunde, das eingangs beschriebene Verbindungsteil bezüglich seiner Justiermöglichkeiten zu verbessern.

Diese Aufgabe wird erfindungsgemäß dadurch gelöst, daß der Flansch ein quadratischer Vierloch-Flansch ist, auf dem das Justierelement exzentrisch angeordnet ist.

Durch Drehung des quadratischen Vierloch-Flansches in 90°-Schritten um seine durch den kreisförmigen Zentrierbund bestimmten Mittelachse ist es nunmehr möglich, zur kompensatorischen Vorpositionierung einen Mittenversatz des Justierelementes in verschiedene Richtungen einzurichten, ohne dabei bereits den richtungsspezifischen Justierbereich nennenswert in Anspruch nehmen zu müssen.

Verschiedene Lösungsmöglichkeiten des erfindungsgemäßen Prinzips sowie die Handhabung werden anhand von Ausführungsbeispielen näher erläutert.

In der Zeichnung sind zwei als Beispiele dienende Ausführungsformen der Erfindung sowie eine zum Stand der Technik gehörende Ausführungsform dargestellt. Es zeigen:
- **Figur 1**: **-** ein Verbindungsteil im Querschnitt;
- **Figur 2**: **-** das Verbindungsteil gemäß **Figur 1** in Draufsicht;
- **Figur 3**: **-** eine abgewandelte Ausführungsform in einer Darstellung gemäß **Figur 1;**
- **Figur 4**: **-** eine Draufsicht der Darstellung gemäß **Figur 3** und
- **Figur 5**: **-** in schematischer Darstellung in Seitenansicht und zum Teil im Längsschnitt eine vorbekannte justierbare Kugelgelenkverbindung zwischen zwei Beinprothesen-Komponenten.

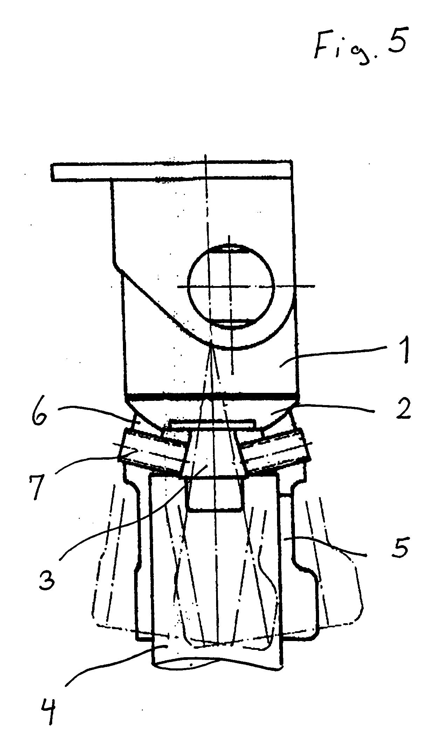

Figur 5 zeigt eine Darstellung aus der vorveröffentlichten deutschen Patentschrift Nr. 19 22 619. An einer Beinprothesen-Komponente 1 ist ein nicht näher dargestellter Flansch befestigt, auf dem ein Justierelement angeordnet ist, das aus einer Kalotte 2 und einer zentrisch auf dieser auf ihre Spitze gestellten vierflächigen Pyramide 3 besteht. An dem in Figur 5 oberen Ende einer zweiten Beinprothesen-Komponente 4 ist eine Buchse 5 befestigt, deren oberes freies Ende als Kugelpfanne 6 ausgebildet ist, die sich auf der Kalotte 2 abstützt und in ihrer Umfangswandung vier um jeweils 90 Umfangsgrad gegeneinander versetzte, mit Innengewinde versehene Bohrungen aufweist, in die jeweils eine Stellschraube 7 eingeschraubt ist, die mit ihrem innenliegenden Ende an der ihr zugeordneten Fläche der vierflächigen Pyramide 3 anliegt.

Durch Lösen einer Stellschraube 7 und entsprechendes Nachspannen der gegenüberliegenden Stellschraube ist eine Verschwenkung des justierbaren Kugelgelenkes in einer definierten Ebene und bei entsprechender Verstellung der beiden anderen Stellschrauben 7 in einer zweiten, senkrecht auf der ersten Verschwenkungsebene stehenden zweiten Verschwenkungsebene möglich. Bei Verstellung der beiden in Figur 5 dargestellten Stellschrauben 7 erfolgt somit eine Verschwenkung der schematisch dargestellten zweiten Beinprothesen-Komponente 4 innerhalb der Zeichenebene gemäß den strichpunktierten Darstellungen. Auf diese Weise läßt sich z. B. die Justierung einer Beinprothese auf die Belastungslinie einstellen. Durch Lösen von zwei benachbarten Stellschrauben 7 ist eine Trennung der beiden Beinprothesen-Komponenten 1,4 möglich, wobei die beiden nicht gelösten Stellschrauben 7 sicherstellen, daß bei einer erneuten Verbindung der beiden Komponenten die zuvor eingestellte Winkelstellung wieder aufgefunden wird. Der Vorteil dieser Konstruktion besteht somit darin, daß eine Winkeljustierung der beiden Beinprothesen-Komponenten 1,4 zueinander durch Verschwenkung in jeweils nur zwei definierten Ebenen erfolgt, wobei sich jede gewünschte Winkelstellung fixieren und auch nach Trennung der beiden Komponenten anschließend wieder auffinden läßt.

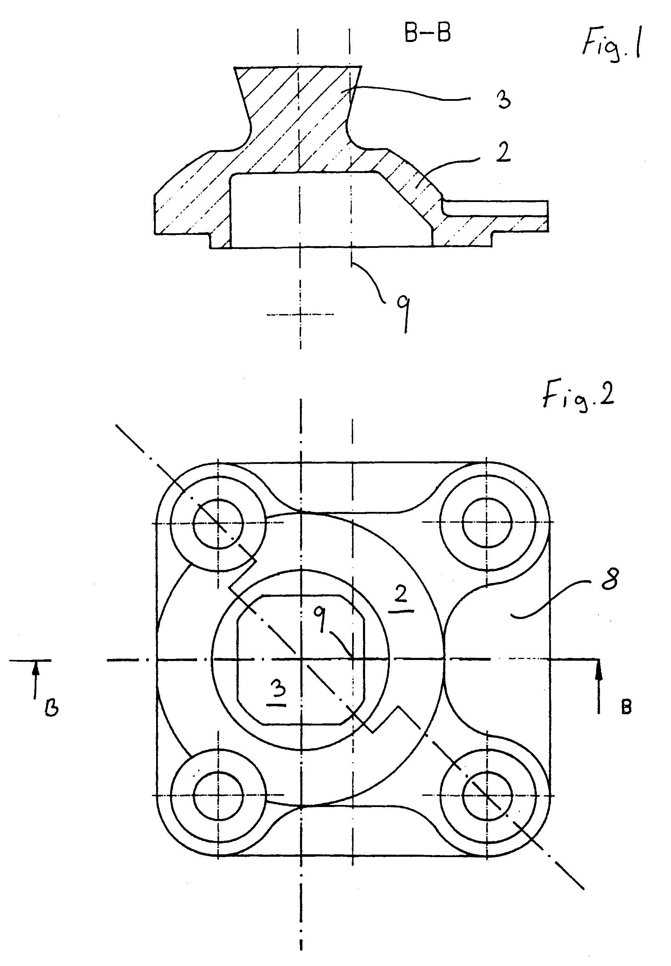

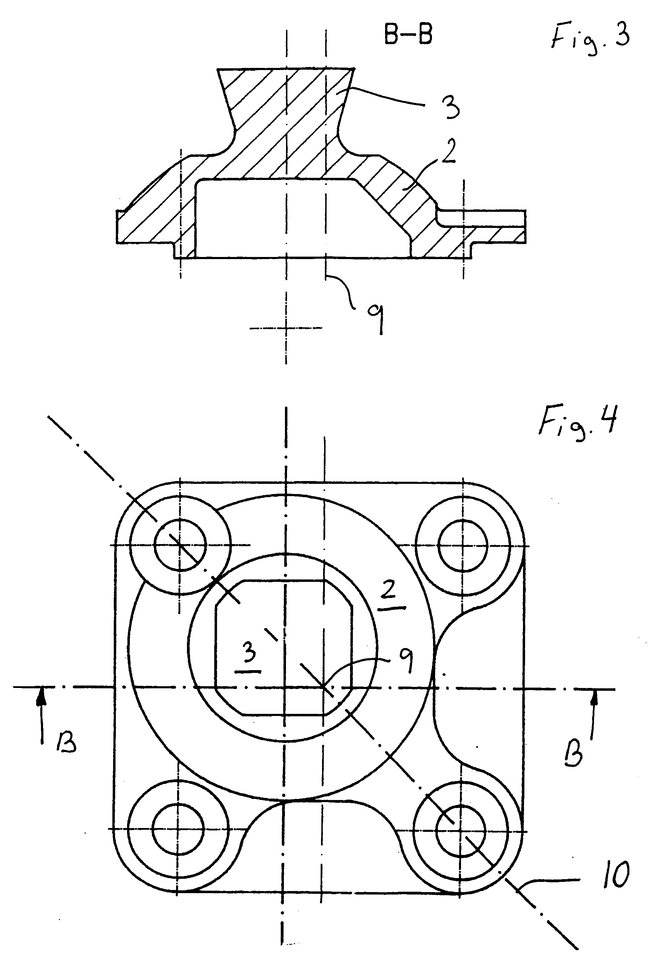

Die Figuren 1 und 2 zeigen eine erfindungsgemäße Ausführungsform für das eine Verbindungsteil des vorstehend beschriebenen Kugelgelenkes. Vorgesehen ist ein quadratischer Vierloch-Flansch 8, auf dem das durch die Kalotte 2 und die Pyramide 3 gebildete Justierelement exzentrisch angeordnet ist. Dies gilt auch für die abgewandelte erfindungsgemäße Lösung gemäß den Figuren 3 und 4.

Bei der Ausführungsform gemäß den Figuren 1 und 2 ist der Mittenversatz des Justierelementes 2,3 gegenüber der durch den kreisförmigen Zentrierbund des Flansches 8 bestimmten Mittelachse 9 auf einer zu je zwei Flanschkanten parallelen Mittellinie vorgenommen worden, während bei der Ausführungsform gemäß den Figuren 3 und 4 der Mittenversatz auf einer der Flansch-Diagonalen 10 erfolgt ist.

Durch Drehung des bei beiden erfindungsgemäßen Ausführungsformen gleichen quadratischen Vierloch-Flansches 8 in 90°-Schritten um seine Mittelachse 9 ist es möglich, zur kompensatorischen Vorpositionierung einen Mittenversatz des Justierelementes 2,3 einzurichten und zwar bei der Ausführungsform gemäß den Figuren 1 und 2 nach vorne, außen, hinten oder innen und bei der Ausführungsform gemäß den Figuren 3 und 4 nach vorne/außen, außen/hinten, hinten/innen und innen/vorne. Dabei ist für die übliche Versorgung vor allem die Einrichtbarkeit der Positionen hinten und innen (Ausführungsform gemäß Figur 1 und 2) bzw. hinten/innen (Ausführungsform gemäß den Figuren 3 und 4) von besonderer Bedeutung.

## Patentansprüche

1. Verbindungsteil eines Kugelgelenkes (2,6) zwischen Beinprothesen-Komponenten (1,4) mit einem zur Befestigung an der einen Komponente (1) bestimmten Flansch, auf dem ein Justierelement (2,3) angeordnet ist, das aus einer Kalotte (2) und einer zentrisch auf dieser auf ihre Spitze gestellten vierflächigen Pyramide (3) besteht, **dadurch gekennzeichnet**, daß der Flansch ein quadratischer Vierloch-Flansch (8) ist, auf dem das Justierelement (2,3) exzentrisch angeordnet ist.

## Claims

1. Connection part of a ball joint (2, 6) between leg prosthesis components (1, 4) having a flange which is intended to be secured to the one component (1) and on which there is arranged an adjusting element (2, 3) which comprises a cap (2) and a four-sided inverted pyramid (3) positioned centrally thereon, characterized in that the flange is a square four-hole flange (8) on which the adjusting element is arranged excentrically.

## Revendications

1. Pièce de raccordement d'une articulation sphérique (2, 6) entre composants (1, 4) de prothèse de jambe, avec une bride destinée à la fixation à un des composants (1), sur laquelle est disposé un élément d'ajustement (2, 3) qui est constitué d'une calotte (2) et d'une pyramide (3) à quatre faces posée par sa pointe sur cette calotte (2), caractérisée en ce que la bride est une bride carrée (8) à quatre trous sur laquelle l'élément d'ajustement (2, 3) est disposé excentriquement.
